# EUROPEAN PATENT APPLICATION

(11) **EP 3 489 240 A1**
(43) Date of publication of application: **29.05.2019**
(21) Application number: 17204208.7
(22) Date of filing: 28.11.2017
(51) Int. Cl.: C07D 487/14, C08G 61/12, H01L 51/00

(54) **IN-SITU CROSS-LINKABLE HOLE TRANSPORTING TRIAZATRUXENE MONOMERS FOR OPTOELECTRONIC DEVICESTR**

(71) Applicant: Ecole Polytechnique Fédérale de Lausanne (EPFL), 1015 Lausanne (CH)
(72) Inventor: Rakstys, Kasparas, 1950 Sion (CH); Huckaba, Aron, 1950 Sion (CH); Nazeeruddin, MD. Khaja, 1024 Ecublens (CH)
(74) Representative: Ganguillet, Cyril

(57) **Abstract**

The invention discloses a polymerizable triazatruxene-based compound of formula (I), an optoelectronic and/or photoelectrochemical device comprising said compound of formula (I) as hole transporting material in hole transport layer under the form of a polymer, and a method of fabrication thereof.

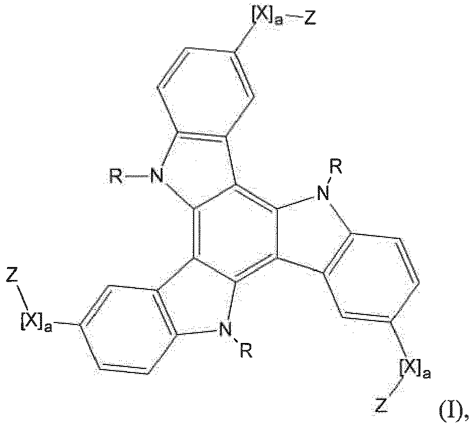

## Description

### Technical Field

The present invention relates to a method for producing an optoelectronic and/or photoelectrochemical device, in particular in a liquid fabrication process, a method for stabilizing one or more underlying layers of the hole transporting layer during liquid fabrication process of an optoelectronic and/or photoelectrochemical device, use of polymerizable triazatruxene-based monomers and triazatruxene-based polymer as hole transporting material, and in particular for stabilizing the one or more underlying layers of the hole transporting layer, an optoelectronic and/or photoelectrochemical device comprising triazatruxene-based polymers as hole transporting material.

### Prior Art and the Problem Underlying the Invention

Nowadays, in order to solve a facing energy problem, various researches for energy that can replace existing fossil fuels, reduce energy consumption and reduce the human activities impacts on the environment have been progressed. Energy is the driving force of the development and progress of human society being important basis for national economic development and the improvement of people's living standards. With the large consumption of traditional fossil energy sources, environmental pollution is worsening and the global energy crisis has become increasingly prominent.

Emerging optoelectronic technologies may provide solutions or important ways to reduce environmental pollution by using devices having reduced energy consumption or converting solar energy, a renewable energy, into electricity. Thus the most promising optoelectronic and/or photoelectrochemical devices which may provide such solutions are organic light emitting diodes (OLEDs) and photovoltaic (PV) devices or solar cell.

OLEDs or devices comprising OLEDs (OLEDs display devices, e.g.) are usually produced by vacuum evaporation or conventional vapor deposition techniques and show good luminance efficiencies. A typical OLED comprises a layer of organic materials situated between two electrodes, the anode and cathode, the whole deposited on a substrate. These organic materials have conductivity levels ranging from insulators to conductors, and are therefore considered organic semiconductors forming emissive and conductive layers. OLEDS may comprise further layers of different materials, hole transport layer (HTL), hole injection layer (HIL), electron transport layer (ETL), electron injection layer (EIL), emissive layer (EML) to improve charge injection at electrodes or block charges (electrons or holes) from reaching the opposite electrode to improve device efficiency.

Although the OLEDs and OLEDs devices have achieved commercial production, there is a large room for technological and economical improvements. A main bottleneck of a large market introduction of OLEDs lies in the high cost levels due the use of capital heavy evaporation facilities. The costs of such devices are currently very high (10,000 Euro per square meter) because such evaporation techniques do not allow a large volume of production without having very high costs and high investment capitals. Further, the use of some materials which may reduce the costs and in the same time improves the device efficiency is impeded by the vapor deposition method, which is very energy intensive at large scales and limits materials selection. Roll-to-roll vapor-deposition methods for organic devices may allow mass production. However, this technique presents drawbacks regarding devices with multiple layers, e.g., due to registration-lining up the different layers to the required degree of accuracy. Although vapor deposition provides well-defined layer structure, this methodology is only applicable to low molecular mass molecules possessing high thermal stability.

Further techniques such as the techniques of liquid fabrication processes and of roll-to-roll solution process have been used to reduce the production costs of OLEDs and OLEDs devices but also to render possible the application of polymers and dendrimers. However, achieving high performance OLEDs by solution-based processes remains a big technological challenge due to possible intermixing of the different active layers in the device stack. Strategies to overcome this, such as using insoluble polymer to solvent and/or dopants, have in the past set upper limits on device performance and lifetime.

Conversion of solar energy to electrical current using thin film in third generation photovoltaic (PV) devices has been widely explored for the last two decades. Current silicon-based solar cell technology is the most mature and widely used device. But the purity of the silicon solar cell materials, the demand for crystal, complex process of fabrication, high production costs are drawbacks to incite the development of low-cost and simple preparation fabrication process of new solar cell and to obtain a solar cell with large scale application. Actually sandwich or monolithic-type PV devices comprising a mesoporous photoanode with an organic or inorganic light harvester, solid-state hole conductor and counter electrode have gained significant interest due to the ease of their fabrication, flexible the selection of materials and cost effective production compared with the current silicon-based solar cell. However, these thin film PV cells generally require a high vacuum manufacturing process, which generally leads to high capital investment and operational expenses.

Because of its unique crystal structure of ABX₃ perovskite materials, said material exhibits long life and high charge mobility, electron and hole carriers, and a large diffusion length. Organic-inorganic perovskites have unique electrical, magnetic, and optical properties, as well as their excellent film processability. Recently, organic-inorganic perovskite absorber under the form of bulk layers of organometallic halide perovskite based on tin (CsSnX₃) or lead (CH₃NH₃PbX₃) have been introduced as semiconducting pigment for light harvesting in PV devices or solar cells, resulting in high power conversion efficiencies (PCE) of PV devices but also in optoelectronic devices such as OLEDs, with perovskite being the emitter or included in the emissive layer.

However, in the PV devices or perovskite solar cells, due to the highly conductive nature of perovskite, a thick layer of hole transporting material (HTM) is required to avoid pinholes. On the other hand, this thicker HTM layer increases the series resistance due to its less conductive nature. Further the use of HTM being free from additive or dopant is also critical for long term stability due to the nature of the perovskite. HTM may be organic or a metal oxide. Attempts were made to find an alternate organic HTM to the most used and usual HTM used in PV devices, namely doped Spiro-OMeTAD (2,2',7,7'-tetrakis(N,N-di-p-methoxyphenyl amine)-9,9-spirobifluorene), since Spiro-OMeTAD shows low stability with perovskite and solid state dye-sensitized devices, probably due to the dopants in Spiro-OMeTAD for increasing the hole mobility. However the use of alternative high-mobility hole conductors hardly competes with the performances equivalent to Spiro-OMeTAD. For example, WO 2010/094636 A1 discloses compounds based on triarylamine used as hole conductors in organic solar cells. Due to its polymeric nature, thus it will induce defects and low device reproducibility. Furthermore polymers are known to be unstable in the low vacuum conditions that follow the step of depositing a cathode in perovskite solar cell.

However, none of these compounds and polymers has shown similar performance than spiro-OMeTAD up to now.

EP 3225624A1 discloses triazatruxene derivatives used as alternative hole conductors in a perovskite solar cell. Said derivatives are used and applied under the form of monomer in a solution. Although PV devices comprising such a triazatruxene derivatives as HTM show good PCE due to the high hydrophobicity acting as a protective layer by retarding the moisture and slowing the degradation of the perovskite, the drawback of pore filing of mesoporous underlying layers of the hole transporting layer is still present as well as pinholes in the thin film. Triazatruxene derivatives were shown to be a good material to be used under the form of hybrids with fluorene as potential blue emitting materials with hole transporting properties for further solution processable blue OLEDs or for fluorescent sensors under the form of conjugated complex polymers (Li et al., J. Meter. Chem. C, 2016, 4, 10574).

US 2010/0308754 A1 further suggests using precursor of polymer or hole transport polymer based on triarylamine derivatives or carbazole derivatives to be tailored to provide the desired electrical and mechanical properties for OLEDs and electronic devices. However said polymers are synthesized before their application in the device. Further said polymers may contain additive useful for crosslinking, unpolymerized polymerizable precursors, a mixture of triarylamine or carbazole derivatives polymerized precursor, and dopants leading to the use of polymers not in pure form but usually in a mixture with additives.

The present invention addresses the disadvantage of organic hole transporting material, when oxidized, which provides instability to the device. It also addresses the use of polymerizable precursors under non-pure form for forming polymer usable as hole transporting material.

The present invention addresses the disadvantage of the instability of the device provided by the presence of the perovskite but also as being exposed to the humidity.

The present invention addresses the disadvantage of the costly fabrication of optoelectronic and/or photoelectrochemical devices and to increase the possibilities of the substrates on which said devices may be fabricated.

The present invention addresses the problems depicted above.

### Summary of the Invention

Remarkably, in some aspects, the present inventors have found that the use of a polymerizable triazatruxene-based monomer, which is in-situ cross-linked or polymerized during the liquid fabrication process, allows the formation of a well-defined layer of HTM, a thin film of HTM without pinholes and good pores filling of the underlying layers, which may be mesoporous, in optoelectronic and/or photoelectrochemical device, the prevention of possible intermixing of the different active layers, the protection of each layer from damage during subsequent material depositions during the production of liquid processed OLEDs and the improvement of power conversion and stability, in particular in solid state solar cell and in perovskite solar cell.

Said use also allows developing cost efficient, roll-to-roll solution process, in particular for optoelectronic devices such as OLEDs, which may be produced directly on flexible substrates allowing new form factors, including their integration in prototype multiple-panel OLED lighting systems and luminaires.

The present invention relates to a triazatruxene-based monomer with polymerizable groups being polymerized in-situ for using as HTM. Said monomers may be further substituted to modulate their highest occupied molecular orbital (HOMO) and the lowest unoccupied molecular orbital (LUMO) of the molecule to match its energy level to the OLED stack. The polymer, being under pure form without additives, is easily obtainable without any purification to the contrary of the synthesis of spiro-OMeTAD.

In an aspect, the invention provides a polymerizable compound of formula (I) 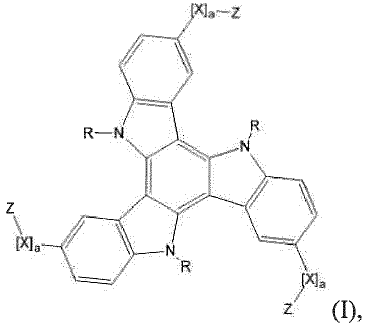 wherein
- R and X are independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said said alkyl, alkenyl, alkynyl, aryl, alkylaryl, alkenylaryl, alkynylaryl, if they comprise 3 or more carbons, may be linear, branched or cyclic, and may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl or C4 to C10 heteroaryl, one or more heteroatoms being selected from N, S and O;
- a is an integer bein 0 or 1,
- Z is independently selected from C2-C10 alkenyl, C2-C10 alkynyl, C4-C20 alkenylaryl, acetylenyl group, alkenyloxy alkyl group, -SH, acrylate group, -OH, R₁C(=O)OH, R₁ being C2-C6 alkenyl, urethane group, ethyl ester group, C4-C20 alkoxyalkenyl, azide group, epoxy compounds, methyl oxirane group, epoxy group, oxiranyl group, and oxetanyl group.

In another aspect, the invention provides an optoelectronic and/or photoelectrochemical device comprising a hole transporting material comprising a polymerizable compound of formula (I) of the invention under polymerized film.

In a further aspect, the invention provides a method for fabricating an optoelectronic and/or photoelectrochemical device comprising a hole transport layer, said method comprising
- providing a layer before providing a hole transport layer, said layer being an underlying layer;
- providing the hole transport layer onto the underlying layer comprising
   - applying a hole transporting material comprising a polymerizable compound of formula (I) of the invention by a liquid deposition process onto the underlying layer, and
   - crosslinking the hole transporting material by thermal, chemical or irradiative means; and
- providing an overlaying layer;
wherein the underlying layer is selected from a hole injection layer, a sensitizer layer, a light-harvester layer, or a conducting current collector; wherein the overlaying layer is selected from an emissive layer when the underlying layer is a hole injection layer, from a counter electrode or a conducting current providing layer when the underlying layer is a sensitizer layer or a light-harvesting layer, or from a sensitizer layer when the underlying layer is a conducting current collector.

Further aspects and preferred embodiments of the invention are detailed herein below and in the appended claims. Further features and advantages of the invention will become apparent to the skilled person from the description of the preferred embodiments given below.

### Brief Description of the Drawings

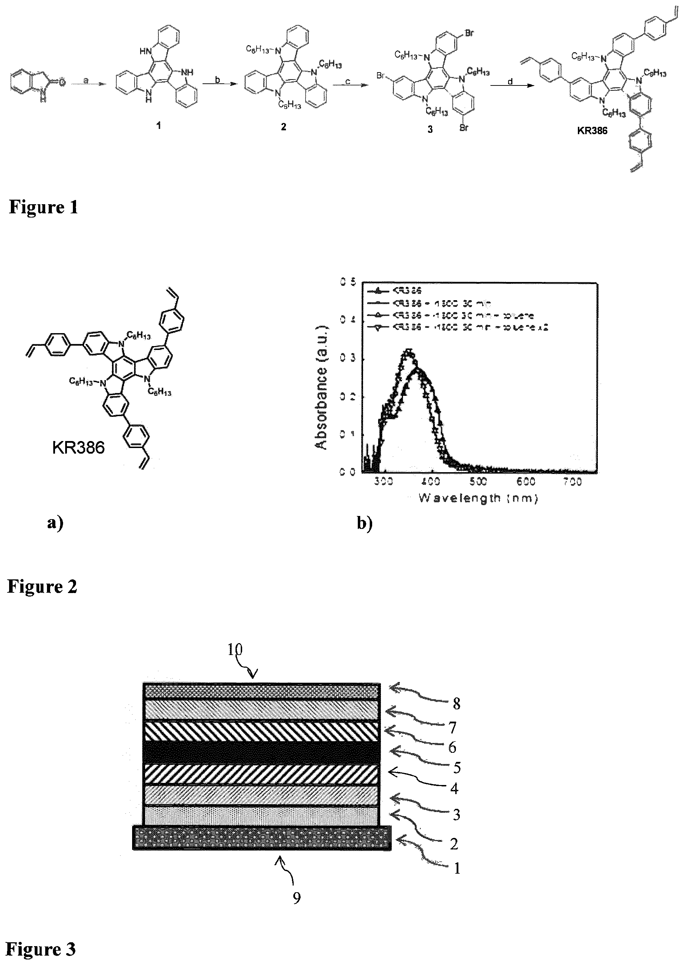

**Figure 1** is a schematic synthesis of a triazatruxene-based compound of the invention KR386. (a) POCl₃, 100 °C; (b) 1-iodohexane, NaH, DMF, 25-120 °C; (c) NBS in DMF, CHCl₃, 0-25 °C; (d) 4-vinylphenylboronic acid, Pd(PPh₃)₄, 2 M aq. K₂CO₃, THF, 80 °C.
**Figure 2 a)** is a polymerizable compound of formula (I) being KR386, and **Figure 2 b)** Absorption spectra of thin film of KR386 under non-polymerized film (red curve), after the thermal crosslinking of thin film of KR386(blue curve) and after washing the polymer thin film by spin-coating (once and twice, respectively pink and orange curves).
**Figure 3** is a schematic structure of an optoelectronic device, OLED according to an embodiment of the invention: substrate layer 1, conductive anode 2, HIL 3, HTL 4, Emissive Layer (EML) 5, ETL 6, EIL, conductive cathode 8, side of light outcoupling 9, and non-emissive side 10.

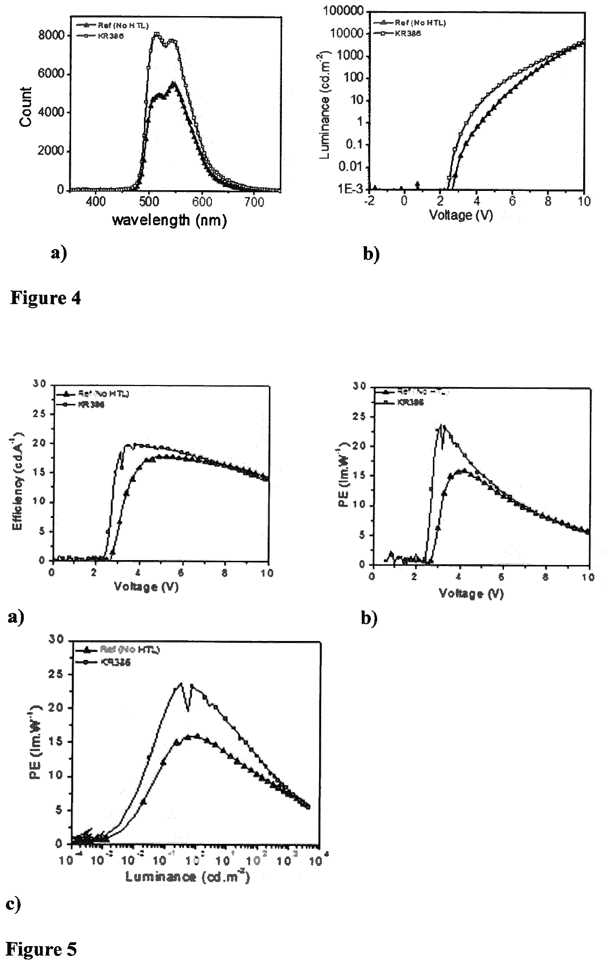

**Figure 4a****)** shows a graphic of luminance intensity of a device being OLED according to the invention with the *in-situ* polymerized hole transporting material KR386 (triangle curve) or without said hole transporting material (empty square curve) and **b)** shows the graphic of Luminance-Voltage characteristics of same device with the *in-situ* polymerized hole transporting material KR386 (triangle curve) or without said hole transporting material (empty square curve).
**Figure 5a****)** shows a graphic of the efficiency of a device being OLED according to the invention with the *in-situ* polymirized hole transporting material KR386 (triangle curve) or without said hole transporting material (empty square curve), **b)** shows a graphic of power conversion efficiency at different driving voltages of same device with the *in-situ* polymirized hole transporting material KR386 (triangle curve) or without said hole transporting material (empty square curve), and **c)** shows a graphic of power conversion efficiency at different current of same device with the *in-situ* polymirized hole transporting material KR386 (triangle curve) or without said hole transporting material (empty square curve)

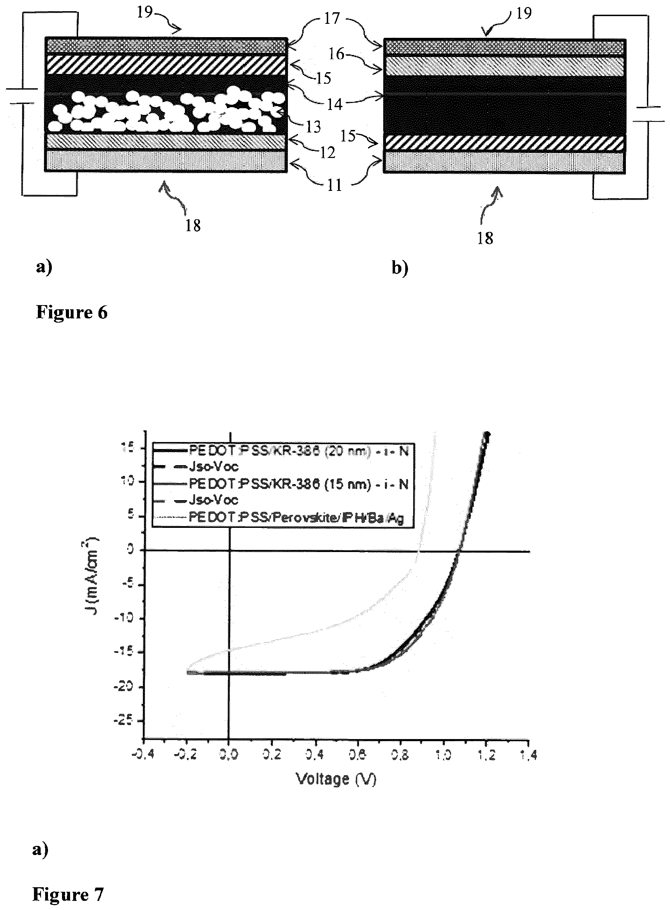

**Figure 6** is schematic structures of optoelectronic and/or photoelectrochemical devices, **a)** n-i-p perovskite solar cell according to an embodiment of the invention: substrate layer 11, conductive anode 12, mesoporous ETL 13, sensitizer (organic-inorganic perovskite) layer 14, hole transport layer HTL 15, conductive cathode 17, direction of incident light 18, and side of the device without direct illumination 19, and **b)** p-i-n perovskite solar cell according to an embodiment of the invention, where layer 16 is ETL.

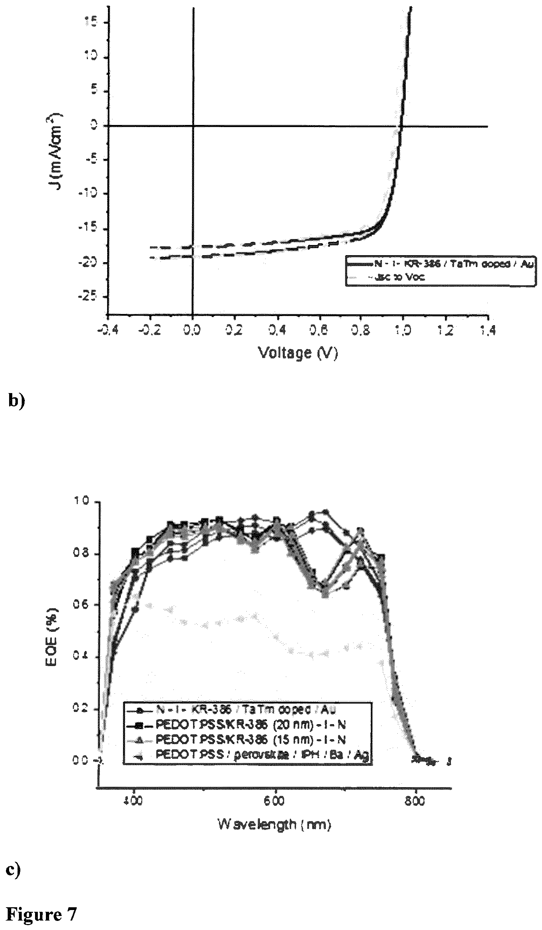

**Figure 7 a)** shows a graphic of Current-Voltage characteristics of p-i-n perovskite solar cells with and without the polymerizable compound of the invention KR386 as hole transporting material (PEDOT:PSS/KR-386 (20nm) black curves, and PEDOT:PSS/KR-386 (20nm) dark grey curves and PEDOT:PSS/Perovskite/IPH/Ba/Ag light grey curves), **b)** shows the graphic of Current-Voltage characteristics of n-i-p perovskite solar cells both devices with and without the polymerizable compound of the invention KR386 as hole transporting material (N-I-P KR386/TaTm black curves) and reverse scan (J_{sc} to V_{oc}) (N-I-P KR386/TaTm light grey curves) and **c)** shows external quantum efficiency of the system. Light gray triangles: pi-n configuration without crosslinkable HTL, Dark gray triangles: p-i-n with crosslinkable HTL, Black square: p-i-n with thicker crosslinkable HTL, Dark gray square, n-i-p with crosslinkable HTM.

### Detailed Description of the Preferred Embodiments

The present invention concerns a polymerizable compound of formula (I) 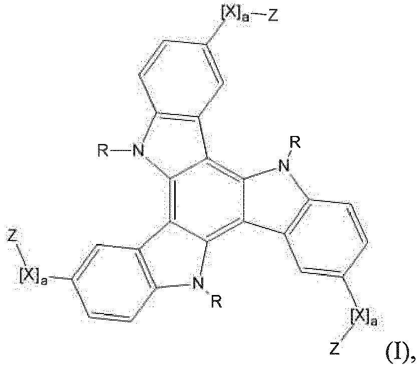 wherein
- R and X are independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said said alkyl, alkenyl, alkynyl moieties, if they comprise 3 or more carbons, may be linear, branched or cyclic, and said alkyl, alkenyl, alkynyl, aryl, alkylaryl, alkenylaryl, alkynylaryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl or C4 to C10 heteroaryl, one or more heteroatoms being selected from N, S and O;
- a is an integer being 0 or 1,
- Z is independently selected from C2-C10 alkenyl, C2-C10 alkynyl, C4-C20 alkenylaryl, acetylenyl group, alkenyloxy alkyl group, -SH, acrylate group, -OH, R₁C(=O)OH, R₁ being C2-C6 alkenyl, urethane group, ethyl ester group, C4-C20 alkoxyalkenyl, azide group, epoxy compounds, methyl oxirane group, epoxy group, oxiranyl group, and oxetanyl group.

R may be independently selected from C1-C10 alkyl, C2-C10 alkenyl, C2-C10 alkynyl, C4-C10 aryl, wherein said said alkyl, alkenyl, alkynyl moieties, if they comprise 3 or more carbons, may be linear, branched or cyclic, and said alkyl, alkenyl, alkynyl, aryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl or C4 to C10 heteroaryl, one or more heteroatoms being selected from N, S and O or by by C1-C10 alkyl, C2-C10 alkenyl, C2-C10 alkynyl, C1-C10 heteroalkyl, C4 to C10 aryl. R may be independently selected from C1-C10 alkyl, C2-C10 alkenyl, C2-C10 alkynyl, C4-C10 aryl, wherein said said alkyl, alkenyl, alkynyl moieties, if they comprise 3 or more carbons, may be linear, branched or cyclic, said moieties being unsubstituted. Preferably R may be independently selected from C1-C6 alkyl, C2-C6 alkenyl, C2-C6 alkynyl, C4-C6 aryl, wherein said said alkyl, alkenyl, alkynyl moieties, if they comprise 3 or more carbons, may be linear, branched or cyclic, said moieties being unsubstituted or substituted as defined herein.

X may be independently selected from C1-C10 alkyl, C2-C10 alkenyl, C2-C10 alkynyl, C4-C10 aryl, wherein said said alkyl, alkenyl, alkynyl moieties, if they comprise 3 or more carbons, may be linear, branched or cyclic, and said alkyl, alkenyl, alkynyl, aryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl or C4 to C10 heteroaryl, one or more heteroatoms being selected from N, S and O or by by C1-C10 alkyl, C2-C10 alkenyl, C2-C10 alkynyl, C1-C10 heteroalkyl, C4 to C10 aryl. X may be independently selected from C1-C10 alkyl, C2-C10 alkenyl, C2-C10 alkynyl, wherein said said alkyl, alkenyl, alkynyl moieties, if they comprise 3 or more carbons, may be linear, branched or cyclic, said moieties being unsubstituted. Preferably X may be independently selected from C1-C6 alkyl, C2-C6 alkenyl, C2-C6 alkynyl, C4-C6 aryl, wherein said said alkyl, alkenyl, alkynyl moieties, if they comprise 3 or more carbons, may be linear, branched or cyclic, said moieties being unsubstituted or substituted as defined herein.

In another embodiment, a of the polymerizable compound of formula (1) is 1. Thus X and/or Z, preferably Z, may be part of the crosslinkable part of the compound of formula (I), if X contains a crosslinkable group.

In a further embodiment, a of the polymerizable compound of formula (I) is 1 and X is selected from C1-C10 alkyl and C4-C10 aryl. Said alkyl and aryl may be substituted or unsubstituted as defined herein. Preferably X may be selected from C1-C6 alkyl and C4-C6 aryl; said alkyl and aryl may be substituted or unsubstituted as defined herein.

Z is the crosslinkable part or functional group of the polymerizable compound of formula (I) with the further polymerizable compounds of formula (I), present in the "in-built" device of the present invention.

Z of the polymerizable compound of formula (I) is independently selected from C2-C6 alkenyl, C2-C6 alkynyl, C4-C12 alkenylaryl, acetylenyl group, alkenyloxy alkyl group, -SH, acrylate group, -OH, R₁C(=O)OH, R₁ being C2-C6 alkenyl, urethane group, ethyl ester group, C4-C20 alkoxyalkenyl, azide group, epoxy compounds, methyl oxirane group, epoxy group, oxiranyl group, and oxetanyl group. Z may be independently selected from C2-C6 alkenyl, C2-C6 alkynyl, C4-C12 alkenylaryl, C2-C12 alkenyloxy alkyl group, -SH, acrylate group, -OH, R₁C(=O)OH, R₁ being C2-C6 alkenyl, urethane group, ethyl ester group, C4-C20 alkoxyalkenyl, azide group, epoxy compounds, methyl oxirane group, epoxy group, oxiranyl group, and oxetanyl group.

In one embodiment, a of the formula (I) of the polymerizable compound of the invention is 0. Therefore only the moiety Z is present in the polymerizable compound of formula (I).

In a further embodiment Z is selected from a moiety according to anyone of formulae (1)-(6) 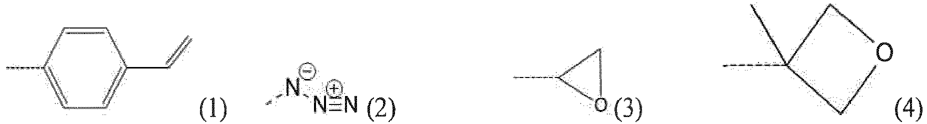 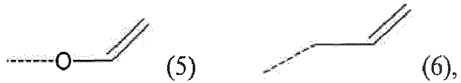 wherein the dotted line represents a single bond between the substituent X of compound of formula (I) or between triazatruxene core of the compound of formula (I), when a is 0, and anyone of the moieties (1)-(5).

The present invention also provides a polymer comprising or consisting of polymerized polymerizable compounds of formula (I) as defined above, wherein the crosslinkable part or moiety Z is crosslinked to another moiety Z of a further compound of formula (I). The compounds of formula (I) in the polymer are identical or a mixture of different compounds of formula (I) with the proviso that the moiety Z is identical between the different compounds of formula (I). The compound of formula (I) represents one unit or a monomer being polymerizable by photo-, thermal or chemical crosslinking to form a polymer during the process of fabrication of the device of the invention. Said polymer is insoluble to the overlying layers and protects from the solubilisation of the underlying layer.

Said polymer comprises a compound of general formula (Ia) 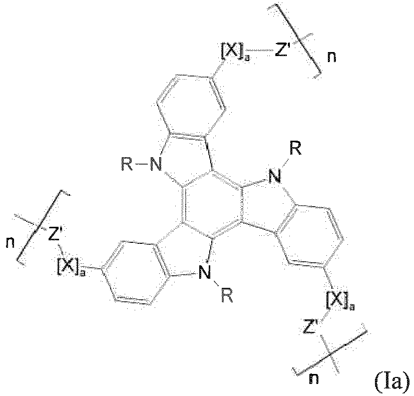 wherein a, R and X are defined herein and as above; n is an integer equal or above 2 or 3; and Z' is selected from a moiety Z under crosslinked form with another moiety Z, being the cross-linkable moiety of the compound of formula (I), of a further compound of formula (I) having the same moiety Z. Preferably n is ≥ 3.

In particular, the moiety 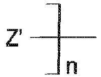 is selected from a moiety according to anyone of formulae (1')-(6') below 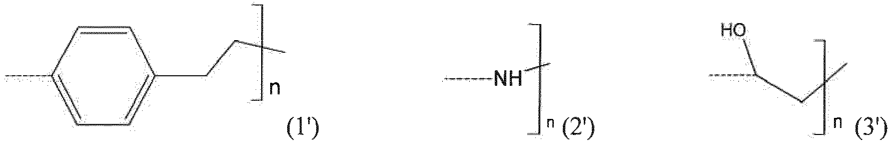 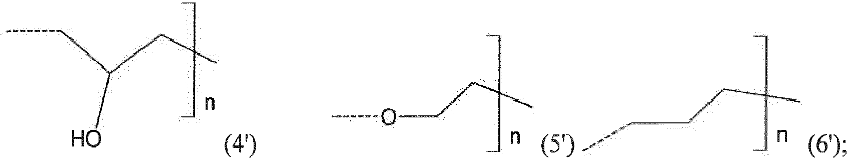 wherein n is defined as above 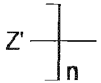 and said moiety is linked to a same moiety 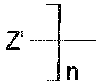 of a further compound of formula (I). Said further compound of formula (I) may be identical or different from the compound of formula (I) to which it is crosslinked, the crosslinkable moiety Z of both or more compounds being the same.

The present invention also provides an optoelectronic and/or photoelectrochemical device comprising a polymerizable compound of formula (I) as defined herein under polymerized film. Said polymerizable compound of formula (I) are under the form of a polymerized film or a polymer comprising or consisting of polymerized or crosslinked polymerizable compounds of formula (I). The crosslinkable part or moiety Z of the polymerizable compound of formula (I), which corresponds to a monomer or a polymerizable unit of a polymer, is crosslinked to another moiety Z of one or more further compounds of formula (I) to form a polymer. The monomers or units in the polymer are linked to each other by modified moieties Z being under crosslinked form such as illustrated by the specific moieties Z' of formulae (1')-(6').

The present invention provides an optoelectronic and/or photoelectrochemical device comprising a hole transporting material, said material comprising a polymerized film of polymerizable compounds of formula (I) as defined herein or a polymer comprising or consisting of polymerized polymerizable compounds of formula (I), wherein the crosslinkable part or moiety Z is crosslinked to another moiety Z as defined above.

The polymerizable compound of formula (I) is used in an optoelectronic and/or photoelectrochemical device as hole transporting material and/or in hole transport layer.

In one embodiment, the optoelectronic and/or photoelectrochemical device comprises a hole transport layer, said hole transport layer comprising a film of hole transporting material of the invention under polymerized form. The optoelectronic and/or photoelectrochemical device of the invention comprises a hole transport layer, said hole transport layer comprising the hole transporting material under a polymerized film. Said film is composed of the polymer obtained by crosslinking the polymerizable compound of formula (I). Said crosslinking or polymerization is performed in-situ, i.e. during the fabrication process of the device. Said film is a continuous film, without pinhole and is insoluble to the solvent or any solution from the underlying and/or overlying layer.

According to another embodiment the hole transport layer has a thickness in the range from 50 to 400 nm. The thickness of the hole transport layer is preferably in the range from 100 to 200 nm, 110 to 190 nm or 120 to 180 nm, and most preferably in the range from 130 to 170 nm, 135 to 165 nm or 140 to 160. In particular, the thickness of the hole transport layer is 150 nm.

For the purpose of the present specification, the expression "in electric contact with" means that electrons or holes can get from one layer to the other layer with which it is in electric contact, at least in one direction. In particular, considering the electron flow in the operating device exposed to electromagnetic radiation, layers through which electrons and/or holes are flowing are considered to be in electric contact. The expression "in electric contact with" does not necessarily mean, and preferably does not mean, that electrons and/or holes can freely move in any direction between the layers. The expression "in electric contact with" does not necessarily mean to directly in electric contact with, but may be in electric contact with through optional layer.

Without to be bound by the theory, the present polymerizable compound of formula (I) may be used as hole transporting material under the form of polymer made during the fabrication process of the device, directly on the device, in any optoelectronic and/or photoelectrochemical device needed such a material to improve the efficiency of the device. In particular said device is an optoelectronic and/or photoelectrochemical device fabricated under liquid process such as spin-coating, meniscus coating, blade coating, screen printing, inkjet printing.

The optoelectronic and/or photoelectrochemical device according to one embodiment is selected from a photovoltaic device, an organic photovoltaic device, a photovoltaic solid state device, a p-i-n heterojunction, a n-i-p heterojunction, a perovskite solar cell, an organic solar cell, a solid state solar cell, a light emitting electrochemical cells and OLED (organic light-emitting diode).

In a particular embodiment, the optoelectronic and/or photoelectrochemical device may be selected from a perovskite solar cell selected from a p-i-n perovskite solar cell and a n-i-p perovskite solar cell, and a light emitting electrochemical cells and OLED.

As illustrated in Figure 3, an organic light-emitting device (OLED) of the invention may comprise a substrate 1 and a conducting layer 2 forming the first electrode or the anode, a hole injection layer (HIL) 3, a hole transport layer (HTL) 4, an emissive layer (EML) 5, an optional electron transport layer (ETL) 6, an electron injection layer (EIL) 7, conducting current providing layer or metallic contact/electrode 8 (cathode). The anode and cathode form the opposite sides: the anode side 9 and the cathode side 10 of said devices.

When a voltage is applied to the anode electrode and the cathode electrode, holes injected from the anode electrode move to the EML, via the HIL and HTL, and electrons injected from the cathode electrode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

The substrate 1 can be any material that can support the organic and metallic layers on it. The material to produce the substrate can be transparent or opaque (e.g., the opaque substrate is used in top-emitting devices) and may be selected from glass, polyethylene terephthalate (PET), quartz, silicon, plastic, or stainless steel. The material preferably forms a thin film which may be flexible. The substrate can be in the form of a sheet or a continuous film. The continuous film can be used, for example, for roll-to-roll manufacturing processes which are particularly suited for plastic, metal, and metallized plastic foils. The substrate can also have transistors or further elements built in to control the operation of an active-matrix OLED device. A single substrate 1 is typically used to construct a larger display containing many pixels (EL devices)

The anode comprises the conducting layer 2 which comprises a material selected from metals, platinum, gold, palladium, and the like; metal oxides, lead oxide, tin oxide, indium-tin-oxide (ITO), and the like; graphite; doped inorganic semiconductors comprising silicon, germanium, gallium arsenide; and doped conducting polymers comprising polyaniline, polypyrrole, polythiophene, and the like.

Said anode further comprises the hole injection layer 3. The material of the hole injection layer (HIL) has good hole conducting properties and is used to effectively inject holes from the conducting layer 2 to the hole transport layer and the emissive layer 5. It also functions as a hole transporting layer. The HIL comprises a material which may be cross-linkable and/or doped. Said material may be selected from aryl-amine type, Poly(N,N'-bis(4-butylphenyl)-N,N'-bis(phenyl) benzidine), Poly((9,9-dihexylfluorenyl-2,7-diyl)-co-(N,N'bis(4- butylphenyl-1,4-phenylenediamine)), poly(9,9-dioctylfluorene)-alt-N, N'-phenyl-1,4-phenylenediamine, poly (2,7-(9,9-di-n-octylfluorene)-(1,4-phenylene-((4 secbutylphenyl)imino)-1,4-phenylene). The dopant material may be conductivity dopants which may reduce the voltage drop along the HIL/HTL and improve the device stability. They may include aromatic amines, e. g. tris(N,N-diphenylamino)triphenylamine, tris(methylphenylphenylamino)triphenylamine. The dopant may be inorganic such as: ferric chloride (FeCl₃), ferric bromide (FeBr₃), antimony pentachloride (SbCl₅), arsenic pentachloride (AsCl₅), boron trifluoride or organic such as 2,3,5,6-tetrafluoro-7,7,8,8-tetracyano-quinodimethane (F4-TCNQ), dicyanodichloroquinone, and trinitrofluorenone. The HIL may be formed by liquid deposition process such us ink jet printing, flex printing, and screen printing.

The hole transport layer 4 comprises the hole transport material of the invention, in particular the polymerizable compound of formula (I). Said polymerizable compound of formula (I) may be applied by liquid deposition method spin-coating, flex printing, ink jet printing screen printing or other deposition method. After the deposition of the hole transporting material, said material is photo, chemical or thermal crosslinked by conventional method to activate the moiety Z of monomers of compound of formula (I) in order to be crosslinked and to form a polymer constituting an insoluble layer being the HTL. Cross-linking can be achieved by exposing the layer (deposited solution) or film or to light, ultraviolet radiation, heat, or by chemical process. This may include the use of ultraviolet curable inks, crosslinkable side chains, crosslinkable chain end groups, monomers which can be cross-linked into polymers, cross-linking agents, initiators, polymer blends, polymer matrices and so on. The general process of cross-linking organic materials is well-known by a person ordinary skilled in the art. Preferably the polymer obtained by the method of the invention is free from additives.

The hole transporting material used in HTL, namely the compound of formula (I) is selected to have to a better or close energy matching with the material of the EML 5 so that holes that are injected can be more easily transported to the EML. It also assists the injection of holes into the EML, reduces exciton quenching, provides better hole transport than electron transport, and block electrons from getting into the HIL and degrading it. The substituted groups are used to modulate the highest occupied molecular (HOMO) and lowest unoccupied orbital (LUMO) of a compound of formula (I) to match the energy level of the OLED stack comprising the HIL and EML.

The EML 5 comprises an organic or organo-metallic material that emits light selected from small molecule light emitting materials and polymer light-emitting materials. The light emitting organic polymers may be selected from polythiophenes, polyphenylenes, polythiophenevinylenes, or poly-p-phenylenevinylenes copolymers, derivatives, or mixtures thereof, polyfluorenes; poly-p-phenylenevinylenes, polytriazatruxenes. The smaller organic molecule material may be selected include tris(8-hydroxyquinolate) aluminum (Alq₃), anthracene, rubrene, tris(2-phenylpyridine) iridium (Ir(ppy)₃), triazine, any metal-chelate compounds and derivatives of any of these materials. Light emitting material may be selected from inorganic or organic-inorganic materials. Said inorganic or organic-inorganic materials are selected from perovskite as defined herein, metal chalcogenide, or any derivative of these materials.

The electron transport layer (ETL) 6, may be selected from small organic molecule materials to include oxadiazoles, triazoles, triazines, imidazoles, thiadiazoles, quinolones, pyridines, quinoxalines, anthrazolines, phenanthrolines, and metal-chelate compounds and derivatives of these materials. The polymer or copolymer ETL materials may be selected from poly oxadiazoles, poly heterocyclic or carbocyclicbased polymers using triazoles, triazines, imidazoles, thiadiazoles, quinolones, pyridines, quinoxalines, anthrazolines, phenanthrolines, and their derivatives.

The electron injection layer (EIL) 7, may be selected from lithium fluoride, cesium carbonate, molybdenum oxide, cesium fluoride, aluminum, rubidium carbonate, rhenium oxide, and their derivatives.

The conducting current providing layer or metallic contact/electrode 8 comprises a material, which can function as a cathode (conductive cathode), being selected from aluminum, indium, silver, gold, magnesium, calcium, lithium fluoride, cesium fluoride, sodium fluoride, and barium, or combinations thereof, or alloys thereof, aluminum, aluminum alloys, and combinations of magnesium and silver or their alloys.

As illustrated in figure 6, an optoelectronic and/or photoelectrochemical device of the invention is a photovoltaic device, a solid state solar cell or a perovskite solar cell. Said perovskite solar cell is selected from a n-i-p perovskite solar cell or a p-i-n perovskite solar cell. In particular, as illustrated in figure 6a, the n-i-p perovskite solar cell may comprise a conducting current collector or substrate 11, n-type semiconductor or conductive anode 12, an optional n-type surface-increasing structure or mesoporous ETL 13, a sensitizer or light-harvesting layer 14, a hole transport layer 15, conducting current providing layer or metallic contact or conductive cathode 17 forming the counter electrode (cathode). As illustrated in figure 6b, the p-i-n perovskite solar cell may comprise a substrate layer 11, a hole transport layer 15, a sensitizer or light-harvesting layer 14, ETL 16, conducting current providing layer or metallic contact 17 forming the counter electrode. Said devices comprise two opposite sides: one side exposed to the light 18 and one device side 19 without direct illumination, towards which either electrons or holes are directed according to the energy levels between the different layers. As illustrated, in the n-i-p perovskite solar cell, the electron are directed to the device side 18 and the holes are directed to device side 19, which creates a potential difference. In the p-i-n perovskite solar cell, the direction of the charges towards the device is inverted compared with the n-i-p perovskite solar cell, i.e. the electron being directed to the device side 19 and the holes being directed to the device side 18.

The conducting current collector or substrate layer 11 is preferably substantially transparent. "Transparent" means transparent to at least a part, preferably a major part of the visible light. Preferably, the conducting support layer is substantially transparent to all wavelengths or types of visible light. Furthermore, the conducting support layer may be transparent to non-visible light, such as UV and IR radiation, for example. The current collector is a conducting support layer providing the support layer of the solar cell of the invention. Preferably, the solar cell is built on said support layer.

According to an embodiment, the solar cell of the invention preferably comprises one or more support layers. The support layer preferably provides the physical support of the device. Furthermore, the support layer preferably provides a protection with respect to physical damage and thus delimits the solar cell with respect to the outside, for example on at least one of the two major sides of the solar cell. The support layer is not specifically shown in the figures. In some embodiments, glass or plastic coated with a TCO current collector is used. These materials, which are commercially available, contain the support as well as the current collector

According to another embodiment, the support of the solar cell may be provided on the side of the counter electrode or the hole selective back contact. In this case, the conductive support layer does not necessarily provide the support of the device, but may simply be or comprise a current collector, for example a metal foil.

The conducting current collector or substrate, collecting the current obtained from the solar cell may comprise a conducting or semiconducting material, such as a conducting organic or inorganic material, such as a metal, doped metal, a conducting metal oxide or doped metal oxide, for example.

The conducting current collector or substrate layer 11 comprises a material selected from indium doped tin oxide (ITO), fluorine doped tin oxide (FTO), ZnO-Ga₂O₃, ZnO-Al₂O₃, tin-oxide, antimony doped tin oxide (ATO), SrGeO₃ and zinc oxide or combinations thereof, which may be coated on a transparent substrate, such as plastic or glass. In this case, the plastic or glass provides the support structure of the layer and the cited conducting material provides the conductivity. Such support layers are generally known as conductive glass and conductive plastic, respectively, which are thus preferred conducting support layers in accordance with the invention.

According to an embodiment, the conducting support layer comprises a conducting transparent layer, which may be selected from conducting glass and from conducting plastic. The conducting current collector is preferably arranged to collect and conduct the current generated in the working electrode or photoanode. In another embodiment, the current collector 11 is in electric contact with the n-type semiconductor layer or conductive anode 12, in particular in the case of a n-i-p solar cell.

The n-type semiconductor layer or conductive anode 12 comprises n-type semiconductor materials selected from n-type metal oxides or n-type semiconducting polymers. The n-type metal oxides may be selected from Ti, Sn, Fe, Zn, W, Mo, Nb, SrTi, Si, Ti, Al, Cr, Sn, Mg, Mn, Zr, Ni, and Cu. The n-type semiconducting polymers may be selected from PCBM-like C60 and fullerene derivatives selected from [6,6]-phenyl-C₆₁-butyric acid methyl ester PCBM-like C60 or PCBM), 1,4,5,8,9,11-hexazatriphenylene-hexacarbonitrile (HAT-CN), (C₆₀-Iₕ)[5,6]fullerene (C60), (C70-D5h)[5,6]fullerene (C70), [6,6]-Phenyl C₇₁ butyric acid methyl ester (PC70BM), 2,9-dimethyl-4,7-diphenyl-1,10-phenanthroline (BCP), 1,3,5-tri(phenyl-2-benzimi-dazolyl)-benzene (TPBI), preferably PCBM, HAT-CN, C60, C70, PC70BM. PCBM-like C60 being a fullerene derivatives means [6,6]-phenyl C₆₁ butyric acid methyl ester.

The n-type semiconductor as defined above may be used as an electron transport material or hole blocking material for hole blocking layer or ETL and may comprise one, two or more n-type metal oxide layers being compact or porous, if two or more layer being present, each n-type metal oxide layer is of a different or identical type or n-type semiconducting polymer. The blocking layer preferably hinders or prevents electron flow in the opposite direction and/or charge recombination.

According to one embodiment, the optoelectronic and/or photoelectrochemical device comprises a ETL16 comprising a n-type semiconducting polymer as defined above, which may be selected from n-type metal oxides semiconductor or from n-type semiconducting polymer.

In an embodiments, the n-type semiconductor layer or hole blocking layer/ETL is applied by one method selected from atomic layer deposition (ALD) and chemical bath deposition (CBD). Preferably, the metal oxide of n-type semiconducting layer is applied by CBD.

According to a further embodiment, the optoelectronic and/or photoelectrochemical device comprises an optional n-type surface-increasing structure or mesoporous ETL 13 or surface-increasing scaffold structure, which is an optional mesostructure in the solid state photovoltaic device. The n-type surface-increasing structure comprises or consists of one or more mesoporous metal oxide layers, each layer comprising a different or identical metal oxide being selected from metal oxide as defined for the n-type semiconductor or n-type semiconducting layer or conductive anode 12, SiO₂, TiO₂, SnO₂, Fe₂O₃, ZnO, WO₃, Nb₂O₅, MoO₃, NiO, SrTiO₃, ZrO₂, and combinations thereof. According to a preferred embodiment, the mesostructure consists of two films of metal oxide selected from TiO₂, ZrO₂ or three films of metal oxide selected from TiO₂, NiO, ZrO₂. Said mesostructure may be prepared by screen printing or spin coating, for example as is conventional for the preparation of porous semiconductor (e.g. TiO₂) layers in DSCs. See for example, Etgar et al. cited above. Nanoporous semiconductor scaffold structures and layers have been disclosed, for example, in EP 0333641 and EP 0606453.

According to a further embodiment, the optoelectronic and/or photoelectrochemical device comprises an optional n-type surface-increasing structure 13 made from non-conducting materials, e.g. from an insulating material. In this case, the sensitizer or light-harvester 14, for example an organic inorganic perovskite pigment, which is deposited on the surface increasing scaffold structure, is also in contact with an n-type semiconductor layer 12. In this case, the surface increasing structure does not continuously cover that n-type semiconductor layer. The surface-increasing structure is preferably structured on a nanoscale and is preferably a mesoporous structure. The structures of said surface increasing structure increase the effective surface compared to the surface of the solar cell.

The nanoparticles comprised in the surface increasing structure preferably have average dimensions and/or sizes in the range of 2 to 300 nm, preferably 3 to 200 nm, even more preferably 4 to 150 nm, and most preferably 5 to 100 nm. "Dimension" or "size" with respect to the nanoparticles means here extensions in any direction of space, preferably the average maximum extension of the nanoparticles.

The invention does not intend to exclude the possibility that there are one or more intermediate layers between the scaffold structure and the conducting current collector support. Such intermediate layers, if present, would preferably be conducting and/or semiconducting.

According to another embodiment, the optoelectronic and/or photoelectrochemical device comprises a sensitizer layer or a light-harvester layer 14.

According to an embodiment, the sensitizer layer or a light-harvester layer of the photoelectrochemical and/or optoelectronic device comprise at least one pigment being selecting from organic, inorganic, organometallic and organic-inorganic pigments or a combination thereof. The thickness of said layer is in the range of 20 to 400 nm, 25 to 300 nm, or 100 to 300 nm, preferably 25 to 400 nm. The sensitizer is preferably a light absorbing compound or material. Preferably, the sensitizer is a pigment, and most preferably the sensitizer is an organic-inorganic pigment.

The sensitizer may, for example, comprise at least one dye disclosed in WO2004/097871A1 or suitable ruthenium dyes are disclosed, for example, in WO2006/010290. The sensitizer may either be a light absorber comprising at least one perovskite material as disclosed in WO 2014/020499A1, WO 2015/087210A1 and/or WO 2016/055025A1.

The sensitizer layer or light-harvester layer may comprise one or more pigments of the group consisting of organometallic sensitizing compounds (phthalocyanine derived compounds, phtalocyanine compounds as disclosed in WO2008/145172A1, porphyrin derived compounds, porphyrin compounds as disclosed in WO2013057538A1), metal free organic sensitizing compounds (diketopyrrolopyrrole (DPP) based sensitizer, sensitizers as recited in WO 2014/033582 A2 and in EP2511924), inorganic sensitizing compounds such as quantum dots, Sb₂S₃ (Antimonysulfide, for example in the form of thin films), aggregates of organic pigments, nanocomposites, in particular organic-inorganic perovskites, and combinations of the aforementioned.

The term "perovskite", for the purpose of this specification, refers to the "perovskite structure" and not specifically to the perovskite material, CaTiO3. For the purpose of this specification, "perovskite" encompasses and preferably relates to any material that has the same type of crystal structure as calcium titanium oxide and of materials in which the bivalent cation is replaced by two separate monovalent cations. The perovskite structure has the general stoichiometry AMX₃, where "A" and "M" are cations and "X" is an anion. The "A" and "M" cations can have a variety of charges and in the original Perovskite mineral (CaTiO₃), the A cation is divalent and the M cation is tetravalent. For the purpose of this invention, the perovskite formulae includes structures having three or four anions, which may be the same or different, and/or one or two organic cations, and/or metal atoms carrying two or three positive charges, in accordance with the formulae presented elsewhere in this specification.

According to an embodiment, the photovoltaic device of the invention comprises one or more layer of an organic-inorganic perovskite. Said organic-inorganic perovskite may be selected from organic-inorganic perovskite described in WO 2014/020499A1, WO 2015/001459A1, WO 2015/107454A1, WO 2015/114521A1, PCT/IB2017/051538, EP 16180656.7.

In an embodiment, the sensitizer layer comprises an organic-inorganic perovskite or a metal halide perovskite according to any one of perovskite-structures of formulae (II), (IIa), (IIb), (IIc), (IId), (IIe), (IIf) and/or (IIg) below:

| | |
|---|---|
| AA'MX₄ | (II) |
| AMX₃ | (IIa) |
| AA'N_{2/3}X₄ | (IIb) |
| AN_{2/3}X₃ | (IIc) |
| BN_{2/3}X₄ | (IId) |
| BMX₄ | (IIe) |
| AA'A₁MX₃ | (IIf) |
| AA₁MX₃ | (IIg) |

wherein,
- A and A' are organic, monovalent cations being independently selected from primary, secondary, tertiary or quaternary organic ammonium compounds, including N-containing heterorings and ring systems, A and A' having independently from 1 to 60 carbons and 1 to 20 heteroatoms;
- A₁ is an inorganic cation selected from Cs⁺, Rb⁺, K⁺, preferably Cs⁺;
- B is an organic, bivalent cation selected from primary, secondary, tertiary or quaternary organic ammonium compounds having from 1 to 60 carbons and 2-20 heteroatoms and having two positively charged nitrogen atoms;
- M is selected from Cu²⁺, Ni²⁺, Co²⁺, Fe²⁺, Mn²⁺, Cr²⁺, Pd²⁺, Cd²⁺, Ge²⁺, Sn²⁺, Pb²⁺, Eu²⁺, Yb²⁺, [SnᵢPb₍₁₋ᵢ₎]⁺, [SnⱼGe₍₁₋ⱼ₎]⁺, and [PbₖGe₍₁₋ₖ₎]⁺, i, j and k being a number between 0.0 and 1.0;
- N is selected from the group of Bi³⁺ and Sb³⁺; and,
- X are independently selected from Cl⁻, Br⁻, I⁻, NCS⁻, CN⁻, NCO⁻, from [I₍₃₋ₘ₎Clₘ]⁻, [I₍₃₋ₙ₎Brₙ]⁻, [Br₍₃₋ᵤ₎Clᵤ]⁻, m, n u being a number between 0.0 and 3.0, and from a combination of two anions selected from Cl⁻, Br⁻, I⁻. In particular, the three or four X may be identical or different. For example, in AMX₃ (formula IIa) may be expressed as formula (IIa') below: AMXiXiiXiii (IIa'), wherein Xi, Xii, Xiii are independently selected from Cl⁻, Br⁻, I⁻, NCS⁻, CN⁻, NCO⁻, from [I₍₃₋ₘ₎Clₘ]⁻, [I₍₃₋ₙ₎Brₙ]⁻, [Br₍₃₋ᵤ₎Clᵤ]⁻, m, n u being a number between 0.0 and 3.0, and from a combination of two anion selected from Cl⁻, Br⁻, I⁻, preferably from halide (Cl⁻, Br⁻, I⁻) and A and M are as defined elsewhere in this specification. Xi, Xii, Xiii may thus be the same or different in this case.

Preferably, if Xi, Xii, Xiii in formulae (IIa), (IIc), (IIf) and (IIg) or Xi, Xii, Xiii, Xiv in formulae (II), (IIb), (IId) or (IIe) comprise different anions X, there are not more than two different anions.

According to perovskite-structure of formula (IIf) or (IIg), A and A' are independently selected from methylammonium cation, formamidinium cations, iodo-carbamimidoyl cation or a combination of said cations.

According to a preferred embodiment, said organic-inorganic perovskite or metal halide perovskite comprises a perovskite-structure according to any one of the formulae (IIh) to (IIm):

| | |
|---|---|
| APbX₃ | (IIh) |
| ASnX₃ | (IIi) |
| ABiX₄ | (IIj) |
| AA'PbX₄ | (IA) |
| AA'SnX₄ | (IIj) |
| BPbX₄ | (IL) |
| BSnX₄ | (IIm) |

wherein A, A', B and X are as defined above in this specification. Preferably, X is preferably selected from Cl⁻, Br⁻ and I⁻, most preferably X is I⁻ or a mixture of Br⁻ and I⁻.

The sensitizer layer comprising organic-inorganic perovskite or metal halide perovskite may comprise a perovskite-structure according to any of the formulae (IIh) to (IIm), more preferably (IIh) and/or (IIi).

According to an embodiment, A and A' are monovalent cations selected independently from any one of the compounds of formulae (20) to (29) below: 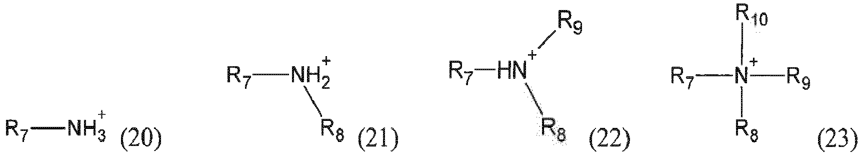 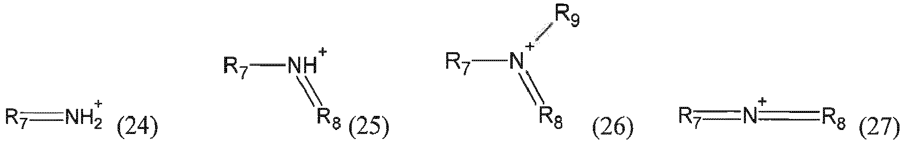 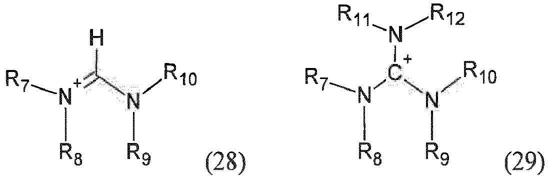 wherein,
R₇, R₈, R₉, R₁₀, R₁₁ and R₁₂ are independently selected from H, C1-C15 organic substituents comprising from 0 to 15 heteroatoms. Counter anion of anyone of formulae (20) to (29), if applicable, may be selected from Cl⁻, Br⁻ or I⁻. According to an embodiment of said C1-C15 organic substituent any one, several or all hydrogens in said substituent may be replaced by halogen and said organic substituent may comprise up to fifteen N, S or O heteroatoms, and wherein, in any one of the compounds (20) to (29), the two or more of substituents present (R₇, R₈, R₉, R₁₀, R₁ and R₁₂, as applicable) may be covalently connected to each other to form a substituted or unsubstituted ring or ring system. Preferably, in a chain of atoms of said C1-C15 organic substituent, any heteroatom is connected to at least one carbon atom. Preferably, neighboring heteroatoms are absent and/or heteroatom-heteroatom bonds are absent in said C1-C15 organic substituent comprising from 0 to 15 heteroatoms. The heteroatoms may be selected from N, S, and/or O. According to an embodiment, R₇, R₈, R₉, R₁₀, R₁₁ and R₁₂ are independently selected from H, C1 to C15 aliphatic and C4 to C15 aromatic or heteroaromatic substituents, wherein any one, several or all hydrogens in said substituent may be replaced by halogen and wherein, in any one of the compounds (20) to (29), the two or more of the substituents present may be covalently connected to each other to form a substituted or unsubstituted ring or ring system.

According to a preferred embodiment, the organic-inorganic perovskite is selected from a compound of formula (II), (IIa), (IIf) or (IIg).

According to an embodiment, B is a bivalent cation selected from any one of the compounds of formulae (49) and (50) below:

R₁₃-G-R₁₄ (49)

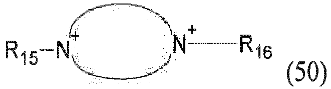

wherein,
in the compound of formula (49), G is an organic linker structure having 1 to 10 carbons and 0 to 5 heteroatoms selected from N, S, and/or O, wherein one or more hydrogen atoms in said G may be replaced by halogen; wherein R₁₃ and R₁₄ are independently selected from a compounds of any one of formulae (20) to (29); and wherein, in the compound of formula (50), the circle containing said two positively charged nitrogen atoms represents a substituted or unsubstituted aromatic ring or ring system comprising 4 to 15 carbon atoms and 2 to 7 heteroatoms or 4 to 10 carbon atoms and 2 to 5 heteroatoms, wherein said nitrogen atoms are ring heteroatoms of said ring or ring system, and wherein the remaining of said heteroatoms may be selected independently from N, O and S and wherein R₁₅ and R₁₆ are independently selected from H and from a compounds of any one of formulae (20) to (29). Halogen atom substituting hydrogen atom totally or partially may also be present in addition to and/or independently of said 2 to 7 heteroatoms.

According to an embodiment, R₇, R₈, R₉, R₁₀, R₁₁ and R₁₂ are independently selected from H, C1 to C10 alkyl, C2 to C10 alkenyl, C2 to C10 alkynyl, C4 to C10 heteroaryl and C6 to C10 aryl, wherein said alkyl, alkenyl, and alkynyl, if they comprise 3 or more carbons, may be linear, branched or cyclic, wherein said heteroaryl and aryl may be substituted or unsubstituted, and wherein several or all hydrogens in R₇, R₈, R₉, R₁₀, R₁₁ and R₁₂ may be replaced by halogen.

A, A' and B may be selected from the exemplary rings or ring systems of formulae (31) and (32) (for A, A') and from (33) to (35) (for B) below: 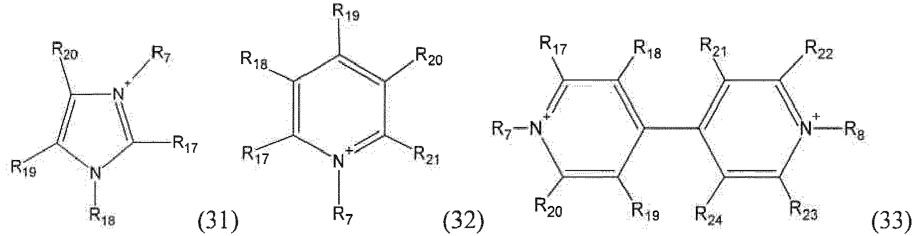 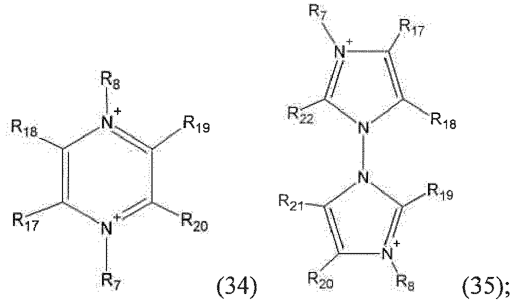 wherein
R₇ and R₈ are selected from substituents as defined above, and R₁₄, R₁₅, R₁₆, R₁₇, R₁₈, R₁₉, R₂₀, R₂₁, R₂₂, R₂₃ and R₂₄ are independently selected from H, halogen and substituents as defined above for R₇, R₈, R₉, R₁₀, R₁₁ and R₁₂. Preferably, R₁₄, R₁₅, R₁₆, R₁₇, R₁₈, R₁₉, R₂₀, R₂₁, R₂₂, R₂₃ and R₂₄ are selected from H and halogen, most preferably H.

According to a preferred embodiment, the metal M is selected from Sn²⁺ and Pb²⁺, preferably Pb²⁺. According to a preferred embodiment, N is Sb³⁺.

According to a preferred embodiment, the three or four X are independently selected from Cl⁻, Br⁻, and I⁻.

The counter electrode and/or metal layer and/or back contact generally comprises a catalytically active material, suitable to provide electrons and/or fill holes towards the inside of the device. The back contact may comprise one or more materials selected from Pt, Au, Ni, Cu, Ag, In, Ru, Pd, Rh, Ir, Os, porous carbon (C), conductive polymer and a combination of two or more of the aforementioned. Conductive polymers may be selected from polymers comprising poly(3,4-ethylenedioxythiophene):poly(styrenesulfonate) (PEDOT:PSS), polyaniline, polypyrrole, polythiophene, polybenzene, polyethylenedioxythiophene, polypropylenedioxy-thiophene, polyacetylene, and combinations of two or more of the aforementioned, for example.

According to another embodiment, the optoelectronic and/or photoelectrochemical device comprises a hole transport layer 15 according to the invention, i.e. comprising a compound of formula (I) being crosslinked or polymerized and forming a film. The hole transport layer is directly or indirectly in electric contact with the sensitizer layer and the positive electrode, i.e. the hole transport layer is between the sensitizer layer and the positive electrode. The positive electrode may be in the side exposed to the light 18 as it is the case for the p-i-n perovskite solar cell illustrated on figure 6b or the "dark" side 19 as it is the case for the n-i-p perovskite solar cell illustrated on figure 6a. The hole transport layer may include further dopants. However, the hole transport layer in the optoelectronic and/or photoelectrochemical device of the invention is free from solvent or other additives.

The hole transport layer HTL 15 comprises a compound of formula (I) as defined herein and for the hole transport layer in the illustrated optoelectronic and/or photoelectrochemical device of Figure 3.

According to one embodiment, the hole transport layer 15 may comprise a further hole transporting material or further layers of hole conductive material different from the hole transporting material of the invention, which may be applied on or before the hole transport layer 15. The further hole conductive material may be selected from poly(3,4-ethylenedioxythiophene):poly(styrenesulfonate) (PEDOT:PSS), poly(3,4-ethylenedioxythiophene):poly(styrenesulfonate):grapheme nanocomposite (PEDOT:PSS:graphene), poly(N-vinylcarbazole) (PVK) and sulfonated poly(diphenylamine) (SPDPA), preferably from PEDOT:PSS, PEDOT:PSS:graphene and PVK, more preferably from PEDOT:PSS. Conductive polymers may also be selected from polymers comprising polyaniline, polypyrrole, polythiophene, polybenzene, polyethylenedioxythiophene, polypropylenedioxy-thiophene, polyacetylene, and combinations of two or more of the aforementioned.

According to another embodiment, the optoelectronic and/or photoelectrochemical device comprises a conducting current providing layer or metallic contact 17 forming the counter electrode. The conducting current providing layer and/or metallic contact and/or back contact comprises a catalytically active material, suitable to provide electrons and/or fill holes towards the inside of the device. The conducting current providing layer and/or metallic contact and/or back contact may comprise one or more materials selected from Pt, Au, Ni, Cu, Ag, In, Ru, Pd, Rh, Ir, Os, porous carbon (C), conductive polymer and a combination of two or more of the aforementioned. Conductive polymers may be selected from polymers comprising poly(3,4-ethylenedioxythiophene):poly(styrenesulfonate) (PEDOT:PSS), polyaniline, polypyrrole, polythiophene, polybenzene, polyethylenedioxythiophene, polypropylenedioxy-thiophene, polyacetylene, and combinations of two or more of the aforementioned, for example.

In a further embodiment, the optoelectronic and/or photoelectrochemical device of the invention comprises a layer underlying the hole transport layer being selected from a hole injection layer, a sensitizer layer, a light-harvester layer, or a conducting current collector, wherein said underlying layer is not cross-linked. Said underlying layer may be the layer beneath/under the hole transport layer or the under layer in electric contact directly with the hole transporting layer or indirectly with the hole transporting layer through an optional layer. The optional layer may be layer blocking a specific type of charge (electron or hole) to avoid any recombination, concentrating the charge or facilitating the flow of a specific charge. The selection of the optional layer depends on the direction of the flow of the electrons or the holes between the two opposite side and their respective polarity, and/or the energy level of the preceding layer of the underlying layer.

According to another embodiment, the optoelectronic and/or photoelectrochemical device of the invention comprises a layer overlying the hole transport layer, said layer being selected from an emissive layer when the underlying layer is a hole injection layer, from a counter electrode or a conducting current providing layer when the underlying layer is a sensitizer layer or a light-harvesting layer, or from a sensitizer layer when the underlying layer is a conducting current collector. Said overlying layer may be the layer above the hole transport layer or the above layer in electric contact directly with the hole transporting layer or indirectly with the hole transporting layer through an optional layer. The optional layer may be layer blocking a type of charge (electron or hole), concentrating the charge or facilitating the flow of a specific charge. The selection of the optional layer depends on the direction of the flow of the electrons or the holes between the two opposite sides and their respective polarity, and/or the energy level of the preceding layer of the underlying layer.

One or more layers may compose the layers overlying or underlying the hole transport layer. Due to the mechanical properties of the film formed by the polymerization of the hole transporting material, the underlying and/or overlying layers are protected against the mixing with the further applied layers during their application by liquid process, against UV light and humidity/moisture, which improves the stability of the device.

In a further aspect, the present invention provide s a method for fabricating an optoelectronic and/or photoelectrochemical device comprising a hole transport layer, said method comprising
- providing a layer before providing a hole transport layer, said layer being an underlying layer;
- providing the hole transport layer onto the underlying layer by liquid deposition comprising
   - applying a hole transporting material comprising a polymerizable compound of formula (I) by a liquid process onto the underlying layer,
   - crosslinking the hole transporting material by thermal, chemical or irradiative means; and
- providing an overlaying layer;
wherein the underlying layer is selected from a hole injection layer, a sensitizer layer, a light-harvester layer, or a conducting current collector; wherein the overlaying layer is selected from an emissive layer when the underlying layer is a hole injection layer, from a conducting current providing layer when the underlying layer is a sensitizer layer or a light-harvesting layer, or from a sensitizer layer when the underlying layer is a conducting current collector. The liquid process to apply the polymerizable compound of formula (I) is selected from spin coating, blade coating, meniscus coating, printing, or spray deposition. The polymerizable compound of formula (I) is preferably under the form of a liquid or a liquid composition.

The method method for fabricating an optoelectronic and/or photoelectrochemical device according to the present invention, comprises further steps of providing further layers underlaying said underlying layer and/or providing further layer overlaying said overlying layer to form a specific optoelectronic and or/photoelectrochemical device, said further layer being selected from substrate layer, conductive anode, ETL, EIL, conductive cathode or metallic contact.

The means of crosslinking the hole transporting material is selected from thermal means such as providing a heating source of temperature up to 180°C e.g., chemical or irradiative means such as providing chemical inducer : azoisobutyronitrile e.g., or irradiative means by providing UV photoirradiation.

According to one embodiment of the method of the invention, the underlying layer is not cross-linked or is not further submitted to a crosslinking before applying the hole transport layer. The underlying layer may be annealed, e.g. to produce the sensitizer but the crosslinking of the underlying layer is not mandatory or required for or before the application of the hole transport layer of the invention. The polymerization in-situ of the hole transport material comprising the polymerizable compound of formula (I) allows to stabilize the underlying layer in the device against environmental harms (UV light, humidity, heat) and harms (layer mixing) during the fabrication process by liquid method.

The crosslinked hole transporting material in the hole transport layer forms a homogeneous polymer film, filling the pore of the underlying layer, if and wherein the hole transport layer does not mix with the liquid material of the overlaying layer during the application of said overlaying layer.

In another embodiment, the optoelectronic and/or photoelectrochemical device obtained by the method of the invention is an organic light-emitting diode, the underlying layer is the hole injection layer and the overlaying layer is the emissive layer.

In a further embodiment, the optoelectronic and/or photoelectrochemical device obtained by the method of the invention is a solid state solar cell having the underlying layer being the sensitizer layer or light-harvesting layer and the overlying layer being the counter electrode or a conducting current providing layer or the underlying layer being a conducting current collector and the overlying layer being a sensitizer layer or a light-harvesting layer.

According to one embodiment, the optoelectronic and/or photoelectrochemical device obtained by the method of the invention is a solid state solar cell, wherein the sensitizer layer or the light-harvesting layer comprises an organic-inorganic perovskite.

The present invention is described more concretely with reference to the following examples, which, however, are not intended to restrict the scope of the invention.

### Examples

**Example 1: Synthesis of polymerizable compound of formula (I) KR386 (****Figure 1****) *10,15-dihydro-5H-diindolo[3,2-a:3',2'-c]carbazole (1).*** A mixture of 2-indolinone (10 g, 75 mmol) and POCl₃ (50 mL) was heated at 100 °C for 8h. Then, the reaction mixture was poured into ice and neutralized carefully with NaOH. After neutralization, the precipitate was filtered to give the crude product as a brown solid. The crude solution in MeOH was absorbed on silica-gel, dried, loaded and eluated through a thick silica-gel pad with a DCM as a mobile phase. After evaporation of eluate at reduced pressure and recrystallization from acetone, pure pale yellow solid was obtained. (5.5 g, 63 %). ¹H NMR (400 MHz, DMSO-d₆) δ 11.86 (s, 3H), 8.66 (d, J = 7.4 Hz, 3H), 7.71 (d, J = 7.9 Hz, 3H), 7.41 - 7.28 (m, 6H). ¹³C NMR (100 MHz, Acetone-d₆) δ 141.0, 136.4, 124.8, 124.5, 121.5, 121.4, 112.9, 103.3. C₂₄H₁₅N₃[M⁺] Exact Mass = 345.1266, MS (ESI-TOF) = 345.1034.

***5,10,15-trihexyl-10,15-dihydro-5H-diindolo[3,2-a:3',2'-c]carbazole (2).*** To a solution of (1) (500 mg, 1.45 mmol, 1 eq.) in DMF (10 mL), NaH (0.1 g, 5.1 mmol, 3.5 eq.) was added at room temperature and stirred for half hour, then 1-iodohexane (1.23 g, 5.8 mmol, 4 eq.) was added via syringe and the mixture was then refluxed for 2h. The cooled mixture was poured into water and extracted with DCM. The organic phase was dried over MgSO₄. The product was isolated off on a silica gel column with 20 % DCM in hexane to give a product as a pale yellow solid (650 mg, 75%). ¹H NMR (400 MHz, Chloroform-d) δ 8.29 (d, J = 8.0 Hz, 3H), 7.64 (d, J = 8.0 Hz, 3H), 7.45 (t, J = 7.4 Hz, 3H), 7.34 (t, J = 7.6 Hz, 3H), 4.92 (m, 6H), 1.99 (p, J = 7.9 Hz, 6H), 1.38 - 1.16 (m, 18H), 0.81 (t, J = 7.1 Hz, 9H). ¹³C NMR (100 MHz, Chloroform-d) δ 13.80, 21.56, 25.70, 29.17, 31.15, 44.76, 107.14, 110.30, 118.79, 119.80, 120.96, 127.47, 137.25, 138.29. C₄₂H₅₁N₃[M⁺] Exact Mass = 597.4083, MS (ESI-QTOF) = 597.4080.

***3,8,13-tribromo-5,10,15-trihexyl-10,15-dihydro-5H-diindolo[3,2-a:3',2'-c]carbazole (3).*** To a solution of (2) (350 mg, 0.58 mmol, 1 eq.) in 30 mL CHCl₃, (320 mg, 1.8 mmol, 3.1 eq.) of NBS in 5 mL DMF was added dropwise via syringe at 0 °C. After addition reaction mixture was stirred for 1h at room temperature. The mixture was extracted with DCM and organic phase was dried over MgSO₄. The product was isolated off on a silica gel column with 10 % DCM in hexane to give a product as a pale-yellow solid (400 mg, 82%). ¹H NMR (400 MHz, Chloroform-d) δ 8.03 (d, J = 8.6 Hz, 3H), 7.71 (s, 3H), 7.42 (d, J = 8.6 Hz, 3H), 4.81 - 4.70 (m, 6H), 1.96 - 1.76 (m, 6H), 1.12 - 1.28 (m, 18H), 0.80 (t, J = 7.0 Hz, 9H). ¹³C NMR (100 Hz, Chloroform-d) δ 125.9, 124.9, 124.2, 124.0, 123.1, 122.7, 113.4, 112.2, 53.7, 47.4, 31.8, 30.4, 26.5, 22.8, 14.2. C₄₂H₄₈Br₃N₃[M⁺] Exact Mass = 831.1398, MS (ESI-QTOF) = 831.1389.

***5,10,15-trihexyl-3,8,13-tris(4-vinylphenyl)-10,15-dihydro-5H-diindolo[3,2-a:3',2'-c]carbazole (KR386).*** To a degassed mixture of (3) (1 g, 1.2 mmol, 1 eq.), 4-vinylphenylboronic acid (0.9 g, 6 mmol, 5 eq.) in THF (25 mL) and 2 M aqueous K₂CO₃ (5 mL), Pd(PPh₃)₄ (300 mg, 20 %) was added under N₂, the resulting solution was heated to 80 °C overnight. After cooling to room temperature, the mixture was poured into water and extracted with DCM. The organic layer was concentrated and the residue was purified by column chromatography on silica gel with 40% DCM in hexane. 650 mg of yellow solid was obtained (59%). ¹H NMR (400 MHz, Tetrahydrofuran-d₈) δ 8.40 (d, J = 8.4 Hz, 3H), 8.01 (s, 3H), 7.85 (d, J = 8.2 Hz, 6H), 7.68 (d, J = 8.4 Hz, 3H), 7.61 (d, J = 8.3 Hz, 6H), 6.85 (dd, J = 17.6, 10.9 Hz, 3H), 5.88 (d, J = 17.7 Hz, 3H), 5.28 (d, J = 11.4 Hz, 3H), 5.17 - 4.99 (m, 6H), 2.00 (p, J = 8.5, 7.8 Hz, 6H), 1.46 - 1.12 (m, 18H), 0.81 (t, J = 7.2 Hz, 9H). C₆₆H₆₉N₃[M⁺] Exact Mass = 903.5491, MS (MALDI-TOF) = 903.614.

The compounds of formula (I) described here are useful as hole transporting materials for optoelectronic devices. The devices are OLEDs or PSCs (perovskite solar cells) with higher stability and performance than those cells without the invention. In-situ polymerization is performed according to the device fabrication process by thermal, chemical, or photochemical means. Comparison of the ultraviolet/visible light absorption (see Figure 2a and b) of thin films made with one of the compounds with the same films after polymerization protocols indicates that in-situ polymerization does indeed occur, and yields an insoluble polymer product with similar absorption characteristics.

**Example 2: A) N-I-P Perovskite Solar Cell Device Fabrication**
First, patterned, fluorine-doped tin-oxide (FTO) coated glass substrates (Tec15, Pilkington) were cleaned by ultrasonication in an alkaline, aqueous washing solution, rinsing with deionized water, ethanol and acetone and 30 min O₃/UV treatment. A TiO₂ compact layer was then deposited on the substrates by aerosol spray pyrolysis at 450 °C using titanium diisopropoxide bis(acetylacetonate) dissolved in ethanol (1:10, volume ratio) as precursor and oxygen as carrier gas. After cooling to room temperature the substrates were treated in an 0.02 M aqueous solution of TiCl₄ for 30 min at 70 °C, rinsed with deionized water and dried at 500 °C during 15 min. The mesoporous TiO₂ layer composed of 20 nm sized particles was deposited by spin-coating at 2000-5000 rpm for 30 s using a commercially available TiO₂ paste (Dyesol 18NRT, Dyesol), diluted in ethanol (1:3, weight ratio). After drying at 120 °C, the TiO₂ films were gradually heated to 500 °C, baked at this temperature for 15 min and cooled to room temperature. The perovskite layers were fabricated using a single step spin-coating procedure reported by Seok et al. For the perovskite precursor solution 508 mg of PbI₂(TCI), 68 mg PbI₂ (TCI), 180.5 mg formamidinium iodide (Dyesol) and 20.7 mg methylammonium bromide (Dyesol) were dissolved in a 1:4 mixture of DMSO:DMF. The perovskite solution was spun at 5000 rpm for 30s using a ramp of 3000rpms-1. 15s prior to the end of the spin-coating sequence, 100 µl chlorobenzene were poured onto the spinning substrate. Afterwards the substrates were transferred onto a heating plate and annealed at 100 °C for 50 min. The hole-transporting material of the present invention was then deposited by spin-coating at 3000 rpm for 30 s. Finally 60 nm of gold were thermally evaporated on top of the device to form the back contact.

**Example 2: B) P-I-N Perovskite Solar Cell Device Fabrication**
Devices were prepared on cleaned Indium-doped tin oxide (ITO) substrates, by spin-coating a thin layer of PEDOT:PSS from the commercial aqueous dispersion (1200rpm 30sec and a short annealing at 150°C result in 70 nm thickness). On top of this layer a thin film of the hole-transporting material of the present invention was then deposited by spin-coating at 3000 rpm for 30 s from a toluene solution (10 mg ml-1, the layer was then annealed at 180 °C for 3 minutes in air. Then the substrates were transferred to a vacuum chamber integrated into an inert glovebox (MBraun, <0.1 ppm 02 and <0.1 ppm H2O) and evacuated to a pressure of 1 × 10-6 mbar. Two ceramic crucibles were filed with CH3NH3I and PbI2 which were heated to 70 and 250°C, respectively. The film thickness was controlled by the PbI2 evaporation at a rate of evaporation of 0.5 Angstrom per second. For the devices using a solution processed fullerene it was deposited using a chlorobenzene solution at a concentration of 10 mg ml-1 in ambient conditions using a spin coater at a speed of 2000 rpm and on top of this a metal electrode consisting of a bi-layer of Barium (10 nm) and Ag (80 nm) was thermally evaporated. For the devices employing C60 that was prepared by thermally evaporation of C₆₀ followed by a codeposited layer of C60 and N1,N4-bis(tri-p-tolylphosphoranylidene)benzene-1,4-diamine (PhIm) (at a ratio of 1:1). The thickness of the two C₆₀ layers were 10 and 40 nm, respectively and the device was finished by thermally evaporating 80 nm of Ag.

**Example 3: Perovskite Solar Cell Device Characterization.** Current-Voltage-Characteristics were recorded by applying an external potential bias to the cell while recording the generated photocurrent with a Keithley model 2400 digital source meter. The light source was a 450 W xenon lamp (Oriel) equipped with a Schott K113 Tempax sunlight filter (Praezisions Glas & Optik GmbH) in order to match the emission spectrum of the lamp to the AM1.5G standard. Incident photon-to- electron conversion efficiency (IPCE) spectra were recorded with a Keithley 2400 Source meter (Keithley) as function of wavelength under a constant white light bias of approximately 5 mW/cm² supplied by a white LED array. The excitation beam coming from a 300 W xenon lamp (ILC Technology) was focused through a Gemini-180 double monochromator (Jobin Yvon Ltd.) and chopped at approximately 2 Hz. All measurements were conducted using a metal aperture to define the active are of the device.

The compound of formula (I) can be used in perovskite solar cells to efficiently transport holes from the perovskite absorber. Whether deposited on top of the perovskite in n-i-p configuration or under in an p-i-n configuration, good charge extraction was observed. In the p-i-n configuration, the addition of the polymerizable material led to a substantial increase in device performance through an increase in current (J_{sc}, voltage at open circuit (V_{oc}), and fill factor (FF). (see Figure 7 and Table 1)

**Table 1: Photovolatic properties of exemplified solar cells**

| Device | | J_{sc}(mA/cm²) | V_{oc}(mV) | FF | PCE (%) |
|---|---|---|---|---|---|
| NIP solar cell | KR386/TaTm | 21.12 | 0.987 | 71% | 14.8 |
| | KR386 | 9.4 | 0.313 | 62% | 1.8 |
| PIN solar cell | PEDOT:PSS/KR-386 (4 mg/ml) | 21 | 1.064 | 61% | 13.55 |
| | PEDOT:PSS/KR-386 (2 mg/ml) | 21 | 1.07 | 63.55% | 14.2 |
| | P-I-N PEDOT:PSS | 14.3 | 0.880 | 44% | 5.5% |

**Example 4: Organic Light Emitting Diode Device Fabrication**
The electroluminescent devices were prepared by using cleaned ITO substrates. The hole-injection layer (HIL), hole-transport layer (HTL), and emissive layer (EML) were spin-coated. The electron transport layer (ETL) and top electrode were thermally evaporated by using an Edwards Auto500 evaporator integrated into a glovebox. First, a 40 nm PEDOT:PSS (HIL) was spin coated (1500 rpm, 60s) and annealed (180 °C, 15 min) in air.. Then the substrates were transferred to an inert atmosphere in which the hole transporting material was spin coated (7000 rpm, 60 s) on top of PEDOT:PSS. To enhance further crosslinking of the hole transport layer, the thin film was annealed up to 180°C for 30 min. Thickness of 20 nm was obtained for the hole transport thin film. The emissive layer consisted of a mixture of 4,4',4"-Tris(carbazol-9-yl)triphenylamine (TcTa), 1,3-Bis[2-(4-tert -butylphenyl)-1,3,4-oxadiazo-5-yl]benzene (OXD-7), and Tris[2-(p - tolyl)pyridine]iridium(III) (Ir(mppy)₃) at 5 wt.-%. was spin-coated (1500rpm, 60s) from chlorobenzene solution (10 mg.ml-1). The thickness of 30 nm was obtained for the EML. Subsequently 40 nm ETL of 1,3-Bis[3,5-di(pyridin-3-yl)phenyl]benzene (BmPyPhB) were thermally evaporated. Finally, the barium (5 nm) and silver (70 nm) top electrode were thermally evaporated.

OLED devices using this material have indicated the invention yields higher performing devices than when the invention was not used. Luminance was increased, which is an important feature of light emitting devices. The "turn-on" voltage was decreased in devices with the in-situ polymerized HTM, which indicated the device worked with less driving voltage.(see Figures 4a and 4b, 5a and 5b). Both the power efficacy and overall device efficiency were greatly improved when the efficiency was used. The driving voltage needed for high efficiency was decreased, and efficiency over a broad range of driving voltages were improved when the invention was used. The power efficacy over a broad range of luminance values (especially low luminance and low voltage) was greatly improved using the invention, which indicated the device including the invention was a much better light generator. (see Figure 5 and Table 2)

**Table 2: Photovolatic properties of exemplified OLEDs**

| Device | With HTL | Without HTL |
|---|---|---|
| Turn-on Voltage (V) | 2.4 | 2.8 |
| V for 100 cd/m² | 5.8 | 7 |
| V for 1000 cd/m² | 8 | 8.5 |
| Power Efficacy at 3 V (lm/W) | 24 | 6 |
| Efficiency at 3 V (cd/A²) | 19 | 7.5 |

Although the present invention has been explained in relation to its preferred embodiment, it is to be understood that many other possible modifications and variations can be made without departing from the scope of the invention as hereinafter claimed.

## Claims

1. A polymerizable compound of formula (I) 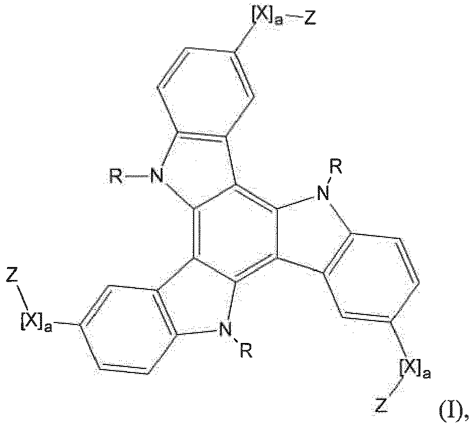 wherein
- R and X are independently selected from C1-C10 alkyl, C2-C10 alkenyl, C2-C10 alkynyl, C4-C10 aryl, C4-C20 alkylaryl, C4-C20 alkenylaryl, and C4-C20 alkynylaryl, wherein said said alkyl, alkenyl, alkynyl moieties, if they comprise 3 or more carbons, may be linear, branched or cyclic, and said alkyl, alkenyl, alkynyl, aryl, alkylaryl, alkenylaryl, alkynylaryl may be unsubstituted or substituted by C1-C10 alkyl, C2-C10 alkenyl, C2-C10 alkynyl, C1-C10 heteroalkyl, C4 to C10 aryl, C2-C10 heteroalkenyl, C2-C10 heteroalkynyl or C4 to C10 heteroaryl, one or more heteroatoms being selected from N, S and O;
- a is an integer been 0 or 1,
- Z is independently selected from C2-C10 alkenyl, C2-C10 alkynyl, C4-C20 alkenylaryl, acetylenyl group, alkenyloxy alkyl group, -SH, acrylate group, -OH, R₁C(=O)OH, R₁ being C2-C6 alkenyl, urethane group, ethyl ester group, C4-C20 alkoxyalkenyl, azide group, epoxy compounds, methyl oxirane group, epoxy group, oxiranyl group, and oxetanyl group.

2. The polymerizable compound of formula (I) according to claim 1, wherein a is 0.

3. The polymerizable compound of formula (I) according to claim 1, wherein a is 1 and X is selected from C1-C10 alkyl and C4-C10 aryl.

4. The polymerizable compound of formula (I) according to anyone of the preceding claims, wherein Z is selected from a moiety according to anyone of formulae (1)-(6) 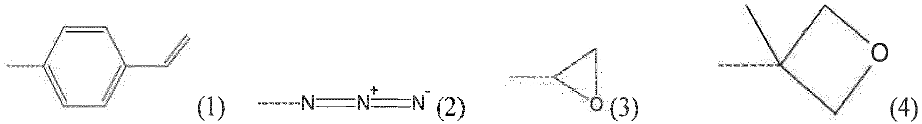 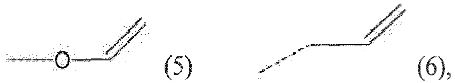 wherein the dotted line represents a single bond between the substituent X of compound of formula (I) or between triazatruxene core of the compound of formula (I), when a is 0, and anyone of the moieties (1)-(5).

5. An optoelectronic and/or photoelectrochemical device comprising a hole transporting material comprising a polymerizable compound of formula (I) as defined in anyone of claims 1-4 under polymerized film.

6. The optoelectronic and/or photoelectrochemical device according to claim 5 comprising a hole transport layer, said hole transport layer comprising a polymerized film of said hole transporting material.

7. The optoelectronic and/or photoelectrochemical device according to anyone of claims 5-6, wherein the hole transport layer has a thickness in the range from 50 to 400 nm.

8. The optoelectronic and/or photoelectrochemical device according to anyone of claims 5-7 comprising a layer underlying the hole transport layer being selected from a hole injection layer, a sensitizer layer, a light-harvester layer, or a conducting current collector, wherein said underlying layer is not cross-linked.

9. The optoelectronic and/or photoelectrochemical device according to anyone of claims 5-8 comprising a layer overlaying the hole transport layer being selected from an emissive layer when the underlying layer is a hole injection layer, from a counter electrode or a conducting current providing layer when the underlying layer is a sensitizer layer or a light-harvesting layer, or from a sensitizer layer when the underlying layer is a conducting current collector.

10. The optoelectronic and/or photoelectrochemical device according to anyone of claims 5-9 is selected from a photovoltaic device, an organic photovoltaic device, a photovoltaic solid state device, a p-i-n heterojunction, a n-i-p heterojunction, an organic solar cell, a solid state solar cell, a perovskite solar cell, a light emitting electrochemical cells and OLED.

11. The optoelectronic and/or photoelectrochemical device according to claim 10 is from a perovskite solar cell selected from a p-i-n perovskite solar cell and a n-i-p perovskite solar cell, and a light emitting electrochemical cells and OLED.

12. A method for fabricating an optoelectronic and/or photoelectrochemical device comprising a hole transport layer, said method comprising
- providing a layer before providing a hole transport layer, said layer being an underlying layer;
- providing the hole transport layer onto the underlying layer comprising
- applying a hole transporting material comprising a polymerizable compound of formula (I) as defined in claims 1-4 by a liquid deposition process onto the underlying layer, and
- crosslinking the hole transporting material by thermal, chemical or irradiative means; and
- providing an overlaying layer;
wherein the underlying layer is selected from a hole injection layer, a sensitizer layer, a light-harvester layer, or a conducting current collector; wherein the overlaying layer is selected from an emissive layer when the underlying layer is a hole injection layer, from a counter electrode or a conducting current providing layer when the underlying layer is a sensitizer layer or a light-harvesting layer, or from a sensitizer layer when the underlying layer is a conducting current collector.

13. The method for fabricating an optoelectronic and/or photoelectrochemical device according to claim 12, wherein said optoelectronic and/or photoelectrochemical device is an organic light-emitting diode, the underlying layer is the hole injection layer and the overlaying layer is the emissive layer.

14. The method for fabricating an optoelectronic and/or photoelectrochemical device according to claim 12, wherein said optoelectronic and/or photoelectrochemical device is a solid state solar cell having the underlying layer being the sensitizer layer or light-harvesting layer and the overlying layer being the counter electrode or a conducting current providing layer or a solid state solar cell having the underlying layer being a conducting current collector and the overlying layer being a sensitizer layer or a light-harvesting layer.

15. The method for fabricating an optoelectronic and/or photoelectrochemical device according to anyone of claims 12-14, wherein the sensitizer layer or the light-harvesting layer or the emissive layer comprises an organic-inorganic perovskite.
